(19) Europäisches Patentamt
European Patent Office
Office européen des brevets

(11) **EP 2 492 605 A1**

(12) **EUROPEAN PATENT APPLICATION**
published in accordance with Art. 153(4) EPC

(43) Date of publication:
**29.08.2012 Bulletin 2012/35**

(21) Application number: **10824834.5**

(22) Date of filing: **13.10.2010**

(51) Int Cl.:
**F24F 11/02** (2006.01)     **F24F 6/00** (2006.01)
**F24F 6/18** (2006.01)

(86) International application number:
**PCT/JP2010/067931**

(87) International publication number:
**WO 2011/048985 (28.04.2011 Gazette 2011/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.10.2009 JP 2009244610**

(71) Applicant: **Hitachi High-Technologies Corporation
Tokyo (JP)**

(72) Inventors:
• **OKUSA Takenori
Hitachinaka-shi
Ibaraki 312-8504 (JP)**
• **WATANABE Setsuo
Fujinomiya-shi
Shizuoka 418-0103 (JP)**

(54) **GAS TEMPERATURE/HUMIDITY REGULATION METHOD AND GAS SUPPLY DEVICE**

(57)     When a gas containing an amount of water vapor equivalent to that contained in the gas at the desired temperature Td and the desired relative humidity Hd is assumed, and the temperature of the gas of when its relative humidity is 100% is defined as an intermediate target temperature Tc, the intermediate target temperature Tc can be calculated from the desired temperature Td and the desired relative humidity Hd. The gas is mixed with water vapor to increase the temperature of the gas to the intermediate target temperature Tc while increasing the relative humidity of the gas to 100%. The gas heated to the intermediate target temperature Tc is heated to the desired temperature Td while maintaining the amount of water vapor contained therein so as to regulate the relative humidity of the gas to the desired relative humidity Hd. Gas maintained at the desired temperature and the desired humidity can thus be stably supplied.

FIG. 1

EP 2 492 605 A1

**Description**

Technical Field

The present invention relates to a method for regulating the temperature and humidity of gas supplied to an apparatus such as a culture apparatus or a chemical analysis apparatus, and also relates to a gas supply device to which the method is applied.

Background Art

In culture apparatuses for culturing cells/tissues, cells/tissues are cultured with the culture chamber (culture room) being humidified in order to minimize evaporation of culture solution. To humidify the culture chamber, in general, a humidification plate containing water is used to naturally vaporize the water, or humidification by bubbling is performed. However, under the existing circumstances, the relative humidity (hereinafter may simply referred to as "humidity") inside the culture chamber is merely kept at a rather high level, and in many cases the humidity is not accurately controlled. Excessive humidification causes dew condensation, whereas insufficient humidification causes evaporation of culture solution. In addition, with respect to chemical analysis apparatuses having a reaction vessel on which a sample is placed, it is desirable that the sample in the reaction vessel be kept at a constant humidity in order to achieve accurate analysis.

In consideration of the above, there has been devised a technique for maintaining gas (e.g., air) supplied to a culture apparatus or the like at a desired temperature and desired humidity. In one example of such technique, the amount of heating applied to gas is adjusted in a manner such that the detection value of a temperature sensor approaches the desired temperature, and the amount of humidification applied to the gas is adjusted in a manner such that the detection value of a humidity sensor approaches the desired humidity (refer to Patent Document 1, etc.).

Prior Art Literature

Patent Documents

Patent Document 1: Patent No. 3094154

Summary of the Invention

Problems to be Solved by the Invention

However, the technique described above has the following problems for which it is difficult to maintain the gas at a desired temperature and humidity.

The first problem is that the change in temperature change is correlated with the change in humidity. Therefore, when both the temperature and humidity of the gas are controlled as in the above-described technique, the controlling becomes difficult. More specifically, in the above technology, gas is humidified by adding water atomized by ultrasonic waves. However in this method, the temperature of the gas may decrease due to the evaporation heat required upon evaporation of the atomized water. If the humidification is done by supplying heated water vapor in order to prevent the temperature from being decreased by the vaporization heat, contrary to the above case, the temperature of the gas increases. On the other hand, if the gas is heated to increase the temperature, the humidity decreases, and if the gas is cooled to decrease the temperature, the humidity increases. Thus, in the above-mentioned technique, the temperature and humidity needs to be controlled while adjusting both the amount of humidification and the amount of heating which influence each other. The control therefore becomes complicated.

The second problem is the excessive decrease in humidity caused by dew condensation. To be more specific, when a gas containing water vapor is cooled by a cooling device (e.g., a heat exchanger for cooling) to decrease the temperature of the gas to the desired temperature, dew condensation occurs on the surface of the cooling device, which may lead to an excessive decrease in humidity. So as to cool down gas to a desired temperature, the temperature of the surface of the cooling device must be lower than the desired temperature. The temperature of the gas near the surface of the cooling device decreases to a temperature lower than the desired temperature, whereby dew condensation occurs. When dew condensation occurs, water vapor may be excessively removed, resulting in the humidity of the gas to decrease to a level much lower than a desired value.

The third problem is the accuracy of the humidity sensor (hygrometer). In general, humidity sensors tend to have low accuracy in comparison with temperature sensors although their performance has been improved. In particular, when humidity close to 100% is to be detected, deterioration in accuracy becomes remarkable. As a hygrometer having relatively high accuracy, a hygrometer which detects humidity using wet and dry temperature difference exists. However, this type of hygrometer has a disadvantage that the response speed is low. Thus, when humidity is controlled based on the detection value of a humidity sensor (especially when gas with high humidity from 80% to 100% is desired to be obtained), it is difficult to expect accurate humidity control.

The present invention has been made taking the above-described problems into consideration. An object of the present invention is to provide a gas temperature/humidity regulation method and a gas supply device which facilitate stable supply of gas maintained at a desired temperature and desired humidity.

Means for Solving the Problems

In order to achieve the above object, an aspect of the present invention provides a gas temperature/humidity regulation method for obtaining a gas regulated at desired temperature and relative humidity, the method comprising the steps of:

calculating an intermediate target temperature from the desired temperature and the desired relative humidity, the intermediate target temperature being, assuming a gas containing an amount of water vapor equivalent to that contained in the gas at the desired temperature and the desired relative humidity, the temperature of the gas when its humidity is 100%;

mixing the gas with water vapor to increase the temperature of the gas to the intermediate target temperature while increasing the relative humidity of the gas to 100%; and

heating the gas heated to the intermediate target temperature to the desired temperature while maintaining the amount of water vapor therein so as to regulate the relative humidity of the gas to the desired relative humidity.

Effects of the Invention

According to the present invention, gas maintained at a desired temperature and desired humidity can be stably supplied.

Brief Description of the Drawings

Fig. 1 is a diagram illustrating the configuration of a culture apparatus provided with a gas supply device according to an embodiment of the present invention, and in addition, transition of the temperature and humidity of air in the gas supply device;

Fig. 2 is a diagram illustrating the configuration of the main controller of the gas supply device according to the embodiment of the present invention;

Fig. 3 is a flowchart illustrating a gas temperature/humidity control flow of the gas supply device according to the embodiment of the present invention;

Fig. 4 is a flowchart illustrating the latter part of the gas temperature/humidity control flow of the gas supply device according to the embodiment of the present invention;

Fig. 5 is a chart illustrating changes in the temperature and humidity of the air and in the output of a vapor heater of when the temperature and humidity control is actually performed by use of the gas supply device according to the embodiment; and

Fig. 6 is a diagram illustrating the configuration of a chemical analysis apparatus provided with the gas supply device according to the embodiment of the present invention.

Modes for Carrying out the Invention

Embodiments of the present invention will be described below with reference to the accompanying drawings.

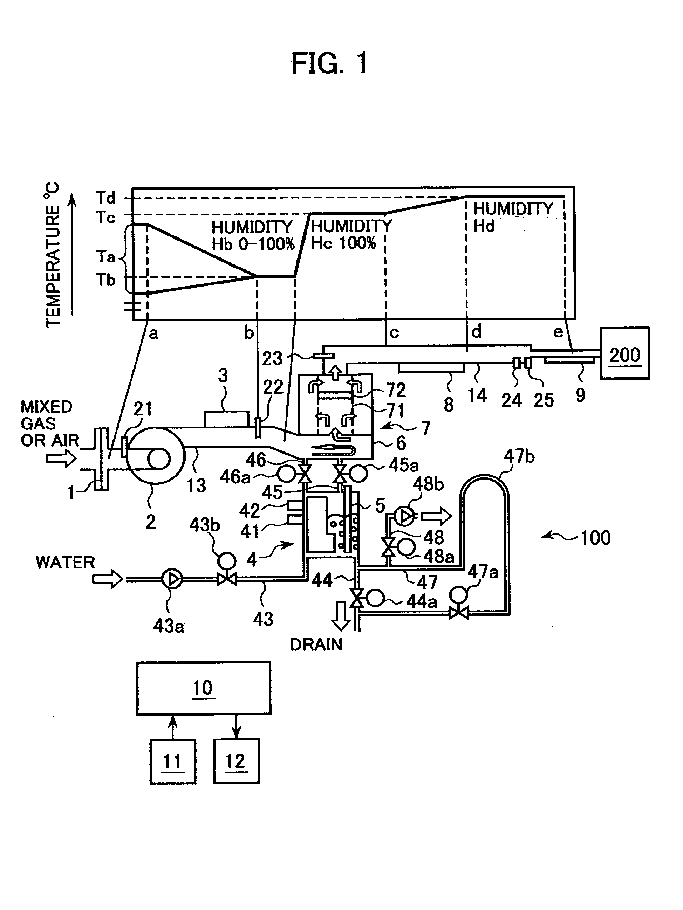

Fig. 1 is a diagram illustrating the configuration of a culture apparatus having a gas supply device according to an embodiment of the present invention. Fig. 1 also shows transition of the temperature and humidity of air in the gas supply device. This embodiment will be described by taking as an example a case where air (gas) having a temperature (Ta) of approximately 15 to 35°C is introduced, and water vapor is added to the air to create air at a desired temperature (final target temperature: Td) and desired humidity (final target humidity: Hd (approximately 70 to 95% in this embodiment)).

The culture apparatus shown in Fig. 1 includes a gas supply device 100 for generating air regulated at the desired temperature Td and the desired humidity Hd, and a culture chamber 200 for culturing cells or the like in the air supplied from the gas supply device 100.

The gas supply device 100 mainly includes a filter 1, a fan 2, a heating/cooling unit 3, a carburetor 4, a vapor heater 5, a moisturizing/mixing unit 6, a moisture removing unit 7, a heat applying heater 8, a temperature maintaining heater 9, a main controller 10, an input unit 11, and a display unit 12.

The fan (gas supply means) 2 supplies air (gas) introduced through the filter 1 to the gas supply device 100. The amount of air supplied to the culture chamber 200 can be adjusted by controlling the number of revolutions of the fan 2. From the standpoint of ensuring enough flow rate and pressure of the air supplied to the culture chamber 200, a turbo fan is desired to be used as the fan 2. The fan 2 of this embodiment is also used as an air supplying means for drying the inside of the gas supply device 100 (the carburetor 4, the moisturizing/mixing unit 6, the moisture removing unit 7 and the like) immediately before operation stop of the gas supply device 100. A temperature sensor 21 for detecting the temperature Ta of the air introduced through the filter 1 is provided between the filter 1 and the fan 2.

The heating/cooling unit (heating and cooling means) 3 is disposed on a gas introducing pipe 13 connecting the fan 2 and the moisturizing/mixing unit 6. The heating/cooling unit 3 heats or cools the air supplied from the fan 2 to regulate its temperature to the initial target temperature Tb. The initial target temperature Tb is an air temperature to which the air is controlled and maintained at before the air is introduced into the moisturizing/mixing unit 6 so that the humidity of the gas can be easily increased to 100% in the moisturizing/mixing unit 6, where the air is heated to an intermediate target temperature Tc (described later) by water vapor generated from the carburetor 4. The initial target temperature Tb is calculated by the main controller 10 (temperature calculation unit 10a) based on the intermediate target temperature Tc, so inevitably the temperature Tb is set at a temperature equal to or lower than the intermediate target temperature Tc. For example, a Peltier device can be used as the heating/cooling unit. Incidentally, when it is understood that the humidity of the air is able to be increased to 100% in the moisturizing/mixing unit 6 without changing the temperature Ta at which the air has been introduced, the temperature of the air do not need to be regulated to the initial target temperature Tb, and therefore the heating/cooling unit 3 may be omitted.

A temperature sensor 22 (second temperature detection means) is provided between the heating/cooling unit 3 and the moisturizing/mixing unit 6. The temperature sensor 22 detects the temperature of the air heated or cooled by the heating/cooling unit 3. The temperature detected by the temperature sensor 22 is used to calculate the output of the heating/cooling unit 3 so as to control the temperature of the air at the inlet of the moisturizing/mixing unit 6 (at the outlet of the heating/cooling unit 3) to the initial target temperature Tb.

The carburetor (vapor generating means) 4 is disposed below the moisturizing/mixing unit 6 to generate water vapor supplied to the moisturizing/mixing unit 6. The carburetor 4 is provided with the vapor heater 5, a first fluid level sensor 41 and a second fluid level sensor 42, and is connected to a feed-water pipe 43, a drain pipe 44, a steam pipe 45 and a dew condensation water pipe 46.

The vapor heater 5 heats water stored in the carburetor 4 to generate water vapor, and its output (the amount of heating) is controlled by the main controller 10. The amount of water vapor generated by the carburetor 4 can be controlled by controlling the output of the vapor heater 5, which in turn controls the temperature of the air at the outlet of the moisturizing/mixing unit 6. The output of the vapor heater 5 in this embodiment is controlled in such a manner that the temperature of the air at the outlet of the moisturizing/mixing unit 6 reaches the intermediate target temperature Tc. Although the details will be described later, the intermediate target temperature Tc represents, assuming air containing an amount of water vapor to be contained in the air at the final target temperature Td and the final target humidity Hd, the temperature of the air when its relative humidity is 100%.

The first fluid level sensor 41 and the second fluid level sensor 42 detect the level of the water surface in the carburetor 4. The sensors 41 and 42 are used to adjust the water quantity (the level of the water surface) in the carburetor 4. The first fluid level sensor 41 is disposed below the second fluid level sensor 42 and is used to determine the water supply timing into the carburetor 4. The second fluid level sensor 42 is used to determine the stop timing of the water supply to the carburetor 4. Optical sensors, for instance, are used as the fluid level sensors 41, 42 in this embodiment. The fluid level sensors 41, 42 turn ON when the water surface level is equal to or higher than their installation height, and turns OFF when the water surface level is below their installation height.

The feed-water pipe 43 is for passing water (makeup water) to the carburetor 4. The feed-water pipe 43 is provided with a feed-water pump 43a and a feed-water valve 43b. The feed-water valve 43b is disposed on the downstream side of the feed-water pump 43a, and is opened when water is to be fed to the carburetor 4.

The drain pipe 44 is for passing drain from the carburetor 4. The drain pipe 44 is provided with a drain valve 44a, which is opened to discharge water inside the carburetor 4 to the outside. In addition, the drain pipe 44 is connected with an overflow pipe 47 bypassing the upstream and downstream sides of the drain valve 44a. When the amount of water in the carburetor 4 exceeds a set value, the excess water is discharged to the outside through a U-shaped section 47b of the overflow pipe 47. An overflow valve 47a is provided on the downstream side of the U-shaped section 47b in the overflow pipe 47. Drainage can be stopped by closing the overflow valve 47a. Further, a vacuum pipe 48 is connected to the upstream side of the U-shaped section 47b of the overflow pipe 47. The vacuum pipe 48 is provided with a vacuum valve 48a and a vacuum pump 48b (pressure reducing means) for reducing the pressure inside the carburetor 4. In order to dry the inside of the gas supply device 100, if a method of using the vacuum pump 48b to dry the inside of the carburetor 4 is employed (vacuum method), the drain valve 44a and overflow valve 47a are closed and the vacuum valve 48a is opened.

The steam pipe 45, through which water vapor generated by the carburetor 4 flows, is connected to the moisturizing/mixing unit 6 disposed above the carburetor 4. The steam pipe 45 is provided with a first drying valve 45a. When the inside of the carburetor 4 is to be dried by the vacuum method, the first drying valve 45a is closed. The dew condensation water pipe 46 is connected to the moisturizing/mixing unit 6 in this embodiment. The dew condensation water pipe 46 introduces into the carburetor 4 excess dew condensation water removed from the air in the moisture removing unit 7. The dew condensation water pipe 46 is provided with a second drying valve 46a. When the inside of the carburetor 4 is to be dried by the vacuum method, the second drying valve 46a is closed.

The moisturizing/mixing unit (mixing means) 6 mixes air from the fan 2 with water vapor from the carburetor 4 (steam pipe 45) to increase the temperature of the air to the intermediate target temperature Tc while increasing the relative humidity of the air to 100%. The moisturizing/mixing unit 6 is disposed between the carburetor 4 and the moisture removing unit 7. The air supplied from the fan 2 is mixed with water vapor by the moisturizing/mixing unit 6 to obtain air at temperature Tc with 100% relative humidity. The conditioned air is then introduced into the moisture removing unit 7. Incidentally, the moisturizing/mixing unit 6 in this embodiment has a generally cylindrical shape with its central axis generally in the vertical direction. The air from the fan 2 is introduced toward a position different from the central axis (for example, a position near the periphery of the cylinder). As the air is introduced, it collides with the wall of the moisturizing/mixing unit 6, having a substantially cylindrical shape, and with water vapor to be mixed to each other. The air rises toward the moisture removing unit 7 while spirally rolling as indicated with an arrow in Fig. 1 and getting mixed with vapor. The mixture of air and water vapor in the moisturizing/mixing unit 6 can thus be accelerated. Incidentally, from the standpoint of accelerating the mixture of air and water vapor, the air from the fan 2 is preferably introduced toward the water vapor outlet in the moisturizing/mixing unit 6 so that the airflow crosses the water vapor flow. In addition, as to accelerate the mixture of air and water vapor, the moisturizing/mixing unit 6 may include a stirring plate to obstruct the airflow.

As described above, the intermediate target temperature Tc represents, assuming air containing an amount of water vapor equivalent to that contained in the air at the final target temperature Td and the final target humidity Hd, the temperature of the air when its relative humidity is 100%. In other words, the intermediate target temperature Tc corresponds to the temperature of air obtained by changing the relative humidity of the air at a desired state (at final target temperature Td and final target humidity Hd) to 100% while maintaining the absolute humidity. Therefore, the intermediate target temperature Tc can be calculated from the final target temperature Td and the final target humidity Hd, and resultantly the temperature Tc is inevitably equal to or lower than the final target temperature Td.

An example of a method of calculating the intermediate target temperature Tc will be described. First, saturation vapor pressure (Px) [PmmHg] at temperature (Tx) [°C] of air at any position (point X (corresponds to point "a", "b", "c" or "d" in Fig. 1)) in the gas supply device 100 can be approximated by the following equation (1). Incidentally, in addition to the equation (1), there are various known approximate equations to express saturation vapor pressure at any temperature. An approximate expression applicable to the present invention is not limited to the equation (1).

[Equation 1]

Saturation vapor pressure:

$$Px = f(Tx) = 10^{\left(8.078 - \frac{1735.74}{Tx + 2354}\right)} \quad \ldots\ldots(1)$$

In addition, the relative humidity (Hx) [%] at any position (point X) in the gas supply device 100 is equivalent to a value found by dividing partial water vapor pressure (px) by the saturation vapor pressure (Px). The partial water vapor pressure (px) at the point X can be represented by the following equation (2).

[Equation 2]

Partial water vapor pressure:

$$px = Px \cdot Hx/100 \quad \ldots\ldots(2)$$

From the equations (1) and (2), the water vapor pressure "px" at the point X can be expressed with Tx and Hx as the following equation (3). Therefore, the water vapor pressures "pd", "pc" at points "d" and "c" in Fig. 1 are represented with the temperatures Td, Tc and the relative humidity Hd, Hc of the air by the following equations (4) and (5).

[Equation 3]
Partial water vapor pressure:

$$px = f(Tx) \cdot Hx/100 \quad \ldots\ldots(3)$$

[Equation 4]
Partial water vapor pressure at point "d":

$$pd = f(Td) \cdot Hd/100 \quad \ldots\ldots(4)$$

[Equation 5]
Partial water vapor pressure at point "c":

$$pc = f(Tc) \cdot Hc/100 \quad \ldots\ldots(5)$$

With respect to the equations (4) and (5), the air is not humidified between the point "c" and "d" in this embodiment so "pc = pd" holds. In addition, Td, Hd are known values since they are the target temperature and the target humidity. The relative humidity at the point "c" is 100% (Hc = 100). Thus, the intermediate target temperature Tc can be found from the equations (4) and (5).

If the intermediate target temperature Tc can be calculated, the initial target temperature Tb can be calculated from the intermediate target temperature Tc. Air at the initial target temperature Tb is mixed with water vapor at a high temperature (100°C) in the moisturizing/mixing unit 6 to form air at temperature Tc having a humidity of 100% (Hc). Therefore, the intermediate target temperature Tc can be expressed using Tb by the following formula (6). Incidentally, in the equation (6), "P" represents the atmospheric pressure, "pac" represents the partial pressure of the air at the point "c", "pc" represents the water vapor pressure at the point "c", "Cw" represents the specific heat of the water vapor, and "Ca" represents the specific heat of the air.

[Equation 6]

$$Tc = Tb\frac{pac}{P} + 100\frac{pc}{P} \cdot \frac{Cw}{Ca} \quad \ldots\ldots(6)$$

Values other than Tb in the equation (6) are known values, so the initial target temperature Tb at the point "b" can be calculated by the equation (6). Incidentally, the reason of conditioning the air to temperature Tb upon introduction into the moisturizing/mixing unit 6 is to allow the air to reach 100% humidity by the moisturizing/mixing unit 6 as it reaches the intermediate target temperature Tc. Therefore, the temperature of the air upon introduction into the moisturizing/ mixing unit 6 may be lower than the calculated Tb if the air is originally at a temperature with which the moisturizing/ mixing unit 6 can surely humidify the air to 100% humidity.

The subject returns to description of the configuration of the gas supply device 100. The moisture removing unit (moisture removal means) 7 is disposed over the moisturizing/mixing unit 6. The moisture removing unit removes excess water vapor from the water vapor added to the air by the moisturizing/mixing unit 6. A net for removing excess moisture (dehydration net for removing excess moisture) 71 is provided inside the moisture removing unit 7. As the air passes through the net 71, the excess moisture in the air is removed. The moisture removing unit 7 in this embodiment is provided with a partition 72 in order to make the air pass through the net 71 for a plurality of times so that the excess moisture in the air is sufficiently removed. The moisture removed by the net 71 flows downward by gravity and returns to the carburetor 4 through the moisturizing/mixing unit 6 and the dew condensation water pipe 46. The air from which the excess moisture has been removed by the net 71 is introduced into a gas supply pipe 14 connected to the upper part of the moisture removing unit 7. At this time, the air is at the temperature Tc and has relative humidity of 100%.

The gas supply pipe 14 is connected to the culture chamber 200, which finally delivers the air kept at the desired temperature (Td) and the desired humidity (Hd) into the culture chamber 200. The gas supply pipe 14 is provided with a temperature sensor 23, a heat applying heater 8, a temperature sensor 24, a pressure sensor 25, and a temperature maintaining heater 9, in this order from the upstream to downstream in the air flow direction.

The temperature sensor 23 (first temperature detection means) is disposed at the outlet of the moisturizing/mixing unit 6 to detect the temperature of the air at the outlet of the moisturizing/mixing unit 6 (inlet of the heat applying heater 8). The temperature detected by the temperature sensor 23 is used to calculate the output of the vapor heater 5 so that the temperature of the air at the outlet of the moisturizing/mixing unit 6 is regulated to the intermediate target temperature Tc.

The heat applying heater 8 heats the air (at the temperature Tc and humidity of 100%) discharged from the moisturizing/mixing unit 6 to the final target temperature Td while maintaining the amount of water vapor therein. As described above, the intermediate target temperature Tc is calculated based on the final target temperature Td and the final target humidity Hd. The air heated to the final target temperature Td by the heat applying heater 8 will be conditioned to reach the final target humidity Hd.

The temperature maintaining heater 9 heats the air that passed through the heat applying heater 8 as appropriate to maintain the temperature of the air heated to the final target temperature Td by the heat applying heater 8. The temperature sensor 24 is disposed downstream of the heat applying heater 8 to detect the temperature of the air at the outlet of the heat applying heater 8. The detection temperature of the temperature sensor 24 is used to control the output of the temperature maintaining heater 9 so that the heater 9 maintains the temperature of the air at the final target temperature Td. The pressure sensor 25 detects the pressure of the air heated by the heat applying heater 8 to measure the air flow rate. Incidentally, an air quantity sensor may be used to measure the air flow rate instead of the pressure sensor 25.

The input unit 11 connected to the main controller 10 is a device through which the operator inputs instructions to the gas supply device 100. The operations performed through the input unit 11 are, for example, turning ON/OFF of the main power supply switch of the gas supply device 100, inputting of the desired temperature Td and the desired humidity Hd, turning ON/OFF of the operation switch for instructing the start/stop of the gas supply operation, and instruction for selecting the drying method for the carburetor 4.

The display unit 12 displays characters and figures for generating gas regulated to the desired temperature Td and the desired humidity Hd. The display unit 12 is connected to the main controller 10. Information displayed on the display unit 12 includes, for example, an input request of the desired temperature Td and humidity Hd, a start request of the gas supply operation, and a selection request of the drying method for the carburetor 4.

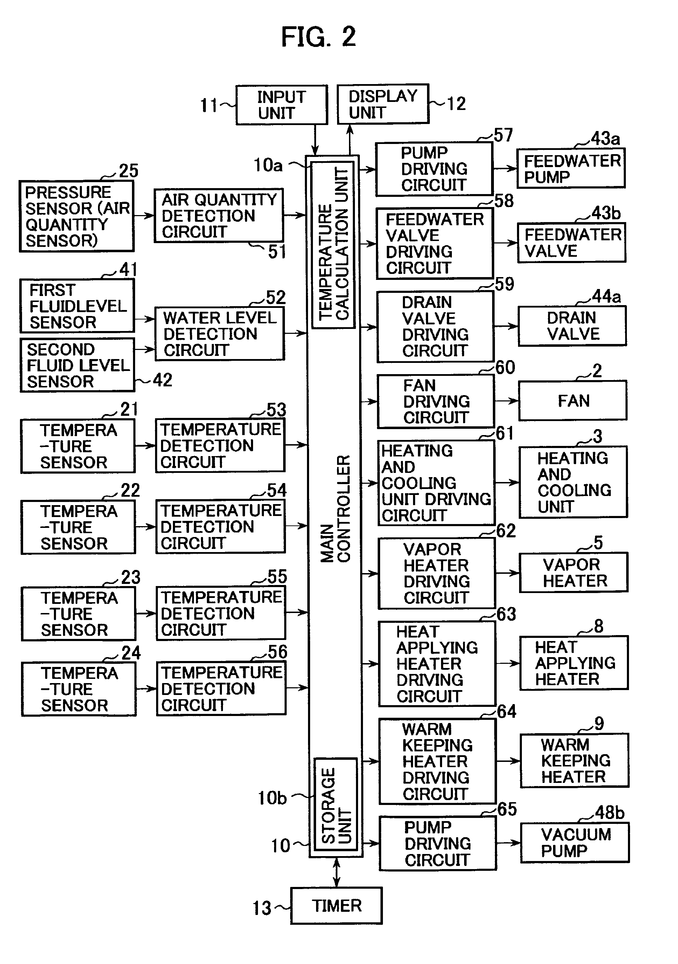

Fig. 2 is a diagram illustrating the configuration of the main controller 10 of the gas supply device according to the embodiment of the present invention. As shown in this figure, the main controller 10 is provided with a temperature calculation unit 10a for calculating the initial target temperature Tb and the intermediate target temperature Tc, and a storage unit 10b for storing calculated values of Tb and Tc. The main controller 10 is connected to the input unit 11, the display unit 12, a timer 13, an air quantity detection circuit 51, a fluid level detection circuit 52, a temperature detection circuit 53, a temperature detection circuit 54, a temperature detection circuit 55, a temperature detection circuit 56, a pump driving circuit 57, a feed-water valve driving circuit 58, a drain valve driving circuit 59, a fan driving circuit 60, a heating/cooling unit driving circuit 61, a vapor heater driving circuit 62, a heat applying heater driving circuit 63, a temperature maintaining heater driving circuit 64, and a pump driving circuit 65.

The timer 13 measures the various time periods required upon controlling of the temperature and humidity of gas. The timing of various control is determined according to the time measured by the timer 13. The air quantity detection circuit 51 is connected to the pressure sensor 25 to detect the air flow rate of the air supplied to the culture chamber 200. The fluid level detection circuit 52 is connected to the fluid level sensors 41, 42 to detect the water level inside the carburetor 4. The temperature detection circuit 53 is connected to the temperature sensor 21 to detect the temperature Ta of the introduced air. The temperature detection circuit 54 is connected to the temperature sensor 22 (second temperature detection means) to detect the temperature of the air heated or cooled by the heating/cooling unit 3. The temperature detection circuit 55 is connected to the temperature sensor 23 (first temperature detection means) to detect the temperature of the air at the outlet of the moisturizing/mixing unit 6. The temperature detection circuit 56 is connected to the temperature sensor 24 to detect the temperature of the air at the outlet of the heat applying heater 8.

The pump driving circuit 57 is connected to the feed-water pump 43a to drive the feed-water pump 43a according to an instruction given by the main controller 10. The feed-water valve driving circuit 58 is connected to the feed-water valve 43b to drive the feed-water valve 43b according to an instruction given by the main controller 10. The drain valve driving circuit 59 is connected to the drain valve 44a to drive the drain valve 44a according to an instruction given by the main controller 10. The fan driving circuit 60 is connected to the fan 2 to drive the fan 2 according to an instruction given by the main controller 10. The heating/cooling unit driving circuit 61 is connected to the heating/cooling unit 3 to drive the heating/cooling unit 3 according to an instruction given by the main controller 10. The vapor heater driving circuit 62 is connected to the vapor heater 5 to drive the vapor heater 5 according to an instruction given by the main controller 10. The heat applying heater driving circuit 63 is connected to the heat applying heater 8 to drive the heat applying heater 8 according to an instruction given by the main controller 10. The temperature maintaining heater driving circuit 64 is connected to the temperature maintaining heater 9 to drive the temperature maintaining heater 9 according to an instruction given by the main controller 10. The pump driving circuit 65 is connected to the vacuum pump 48b to drive the vacuum pump 48b according to an instruction given by the main controller 10.

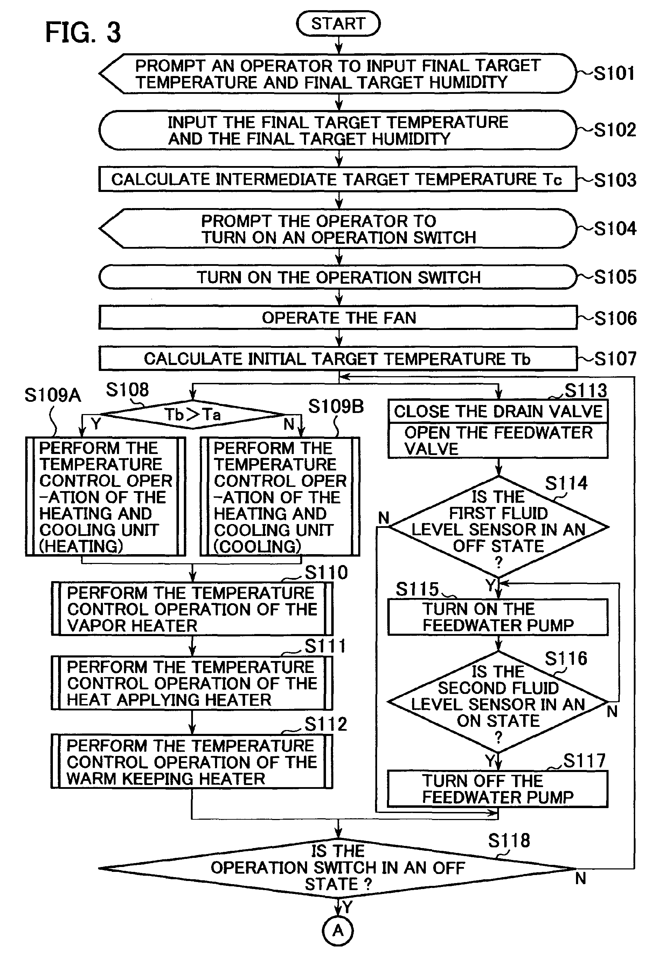

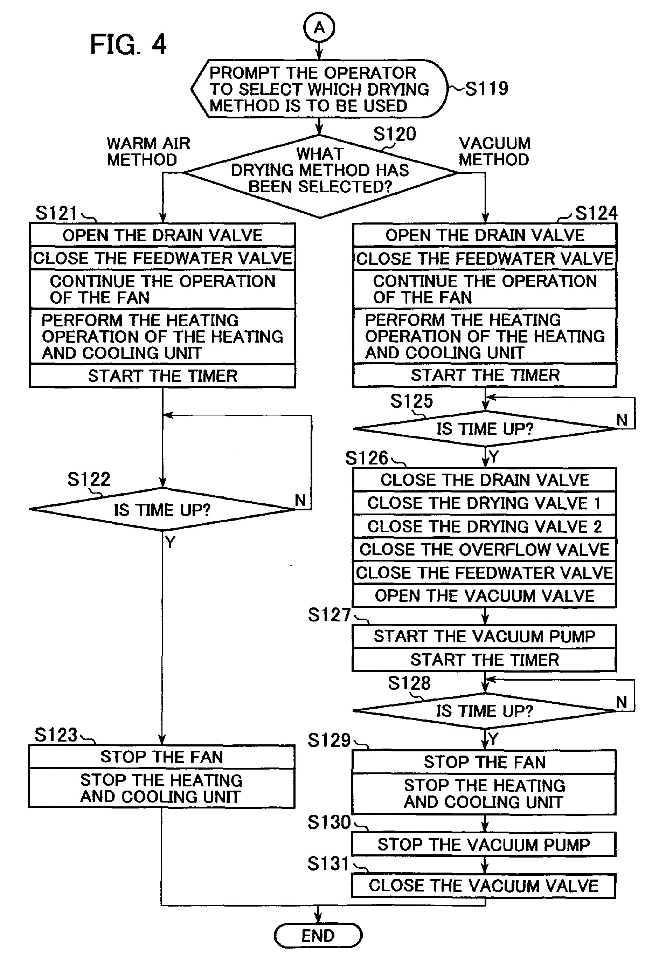

Figs. 3 and 4 show the gas temperature/humidity control flow of the gas supply device 100 configured as above according to the embodiment of the present invention.

When the main power supply switch is turned ON, the gas temperature/humidity control flow shown in the figure starts. The main controller 10 displays a message on the display unit 12 to prompt the operator to input the final target temperature $Td$ and the final target humidity $Hd$ (the desired temperature and the desired humidity) of the air supplied to the culture chamber 200 (S101). As the operator inputs the final target temperature $Td$ and the final target humidity $Hd$ through the input unit 11 (S102), the main controller 10 controls the temperature calculation unit 10a to calculate the intermediate target temperature $Tc$ based on the temperature $Td$ and the humidity $Hd$ inputted in S102, and then stores the intermediate target temperature $Tc$ in the storage unit 10b (S103). The gas supply device 100 will then be prepared to perform the gas supply operation of supplying gas to the culture chamber 200. The main controller 10 displays a message on the display unit 12 to prompt the operator to turn on the operation switch (S104).

As the operator turns on the operation switch (S105), the main controller 10 drives the fan 2 to start the introduction of air (S106). The main controller 10 also calculates the initial target temperature $Tb$ based on the intermediate target temperature $Tc$ calculated in S103, and then stores the initial target temperature $Tb$ in the storage unit 10b (S107).

As the initial target temperature $Tb$ is calculated, the main controller 10 determines whether or not the temperature $Ta$ detected by the temperature sensor 21 is lower than the initial target temperature $Tb$ calculated in S107 (S108). When it is determined in S108 that $Ta$ is lower than $Tb$, the main controller 10 operates the heating/cooling unit 3 in the heating mode, and controls its output so that the temperature detected by the temperature sensor 22 nears the initial target temperature $Tb$. The temperature of the air introduced into the moisturizing/mixing unit 6 is thus controlled (S109A). On the other hand, when it is determined in S108 that $Ta$ is equal to or higher than $Tb$, the main controller 10 operates the heating/cooling unit 3 in the cooling mode, and as with the heating mode, controls its output so that the temperature of the air nears $Tb$, thereby controlling the temperature of the air introduced into the moisturizing/mixing unit 6 (S109B).

The main controller 10 also adjusts the output of the vapor heater 5 so that the temperature detected by the temperature sensor 23 becomes the intermediate target temperature $Tc$, thereby controlling the temperature of the air passed through the moisturizing/mixing unit 6 and the moisture removing unit 7. As the output of the vapor heater 5 is controlled in such manner, an amount of water vapor corresponding to the output of the vapor heater 5 is supplied to the moisturizing/mixing unit 6. The water vapor is mixed with the air that has been conditioned to the initial target temperature $Tb$ in S109A and S109B. The temperature of the air at the outlet of the moisture removing unit 7 increases to $Tc$ while its relative humidity increases to 100% (S110).

The main controller 10 controls the output of the heat applying heater 8 so that the temperature of the air discharged from the moisture removing unit 7 becomes the final target temperature $Td$. The temperature of the air is regulated to the final target temperature $Td$ while the amount of water vapor in the air is maintained, whereby the relative humidity of the air is regulated to the final target humidity $Hd$. That is to say, after the air is mixed with the water vapor by the moisturizing/mixing unit 6, air at a desired temperature $Td$ and desired humidity $Hd$ can be created by only checking its temperature (S111). Incidentally, the output of the heat applying heater 8 can be calculated based on, for example, the difference between the intermediate target temperature $Tc$ and the final target temperature $Td$. The output may as well be adjusted by using the temperature detected by the temperature sensor 24.

The main controller 10 then adjusts the output of the temperature maintaining heater 9 based on the temperature detected by the temperature sensor 24 so that the temperature of the air running through the gas supply pipe 14 is kept at the final target temperature $Td$. Air kept at the final target temperature $Td$ and the final target humidity $Hd$ can thus be supplied to the culture chamber 200 (S112).

Incidentally, in parallel with the series of processing of S108 to S112, the main controller 10 performs the water quantity adjustment control of the water quantity inside the carburetor 4. To be more specific, the drain valve 44a is closed and the feed-water valve 43a is opened to prepare a state in which water can be supplied to the carburetor 4 (S113). Then, whether or not the first fluid level sensor 41 is OFF is determined (S114). When it is determined in S114 that the first fluid level sensor 41 is OFF, the water surface level in the carburetor 4 can be determined to be at below the first fluid level sensor 41. The feed-water pump 43a is driven to start water supply to the carburetor 4 (S115). Subsequently, whether or not the second fluid level sensor 42 is ON is determined (S116). When it is determined that the second fluid level sensor 42 is ON, the feed-water pump 43a is stopped (S117). When it is determined in S114 that the first fluid level sensor 41 is ON, the main controller 10 waits with the state of S113 maintained.

During the series of processing of S108 to S117, the main controller 10 determines whether or not the operation switch has been turned OFF (S118). While the operation switch is kept ON, the main controller 10 repeats the temperature control processing of S108 to S112 and the water quantity adjustment processing of S113 to S117. Meanwhile, when the operation switch is turned OFF, process proceeds to the drying process for drying the inside of the gas supply device 100. After proceeding to the dry process, the main controller 10 displays a message on the display unit 12 to prompt the operator to select which drying method (either a warm air method or a vacuum method) is to be used (S119). The main controller 10 determines which drying method has been selected by the operator through the input unit 11 (S120). Incidentally, the timing of selecting drying method does not need to be after the operation switch is turned OFF as described above. The drying method may be selected beforehand.

When it is determined in S120 that the warm air method is selected, the main controller 10 opens the drain valve 44a, closes the feed-water valve 43b, continues the operation of the fan 2, operates the heating/cooling unit 3 in the heating mode, and activates the timer 13 to start measuring time (S121). The water inside the carburetor 4 is discharged to the outside to empty the carburetor 4, and air heated by the heating/cooling unit 3 will be introduced into the gas supply device 100. Residual water in the carburetor 4, the moisturizing/mixing unit 6, the moisture removing unit 7 and the like evaporates and dries outs, preventing mold and microbes from proliferating therein. During this process, the main controller 10 is performing determination of whether or not the time measured by the timer 13 has reached the predetermined time (set value) required for drying by the warm air method (S122). The main controller 10 stops the fan 2 and the heating/cooling unit 3 at the timing the predetermined time has elapsed (S123), and then stops the operation of the gas supply device 100.

Meanwhile, when it is determined in S120 that the vacuum method has been selected, the main controller 10 first operates to dry the carburetor 4, the moisturizing/mixing unit 6, the moisture removing unit 7 and the like similarly to S121 and S122. The main controller 10 opens the drain valve 44a, closes the feed-water valve 43b, continues the operation of the fan 2, and drives the heating/cooling unit 3 in the heating mode (S124). Subsequently, when the time measured by the timer 13 reaches the set time (S125), the main controller 10 closes the drain valve 44a, the first drying valve 45a, the second drying valve 46a, the overflow valve 47a and the feed-water valve 43b, and opens the vacuum valve 48a (S126). The main controller 10 then starts the vacuum pump 48b, and operates the timer 13 again to start drying the inside of the carburetor 4 by the vacuum method (S127). This causes the internal pressure inside the carburetor 4, the overflow pipe 47 and the like to be low. The residual water vaporizes and dries, preventing mold and microbes from proliferating in the devices. Incidentally, upon this process, it is more effective to warm the area around the carburetor 4 by energizing the vapor heater 5. As the time measured by the timer 13 reaches the set time (S128), the main controller 10 stops the fan 2 and the heating/cooling unit 3 to stop supplying warm air to the gas supply device 100 (S129). Further, the main controller 10 stops the vacuum pump 48b (S130), closes the vacuum valve 48a (S131), and stops the operation of the gas supply device 100.

As described above, the gas supply device according to this embodiment calculates the intermediate target temperature Tc from the final target temperature Td and the final target humidity Hd. Gas is mixed with water vapor to increase the temperature of the gas to the intermediate target temperature Tc while increasing its relative humidity to 100%. The gas heated to the intermediate target temperature Tc is then heated to the final target temperature Td while maintaining the amount of the water vapor therein, thereby conditioning the relative humidity thereof to the final target humidity Hd. The features of this gas supply device are: (A) the control of the temperature and humidity is performed based on only the output from the temperature sensors; (B) output from a humidity sensor (hygrometer, etc.) is not used for the control; and (C) the air is not cooled at all after humidification.

After conditioning the gas to reach the intermediate target temperature Tc and 100% humidity by mixing the gas with water vapor, if the temperature of the gas is increased to the final target temperature Td, the humidity of the gas can be regulated to the final target humidity Hd without depending on a humidity sensor. This enables generation of a gas with more accurate humidity compared to cases where a humidity sensor is used. Therefore, according to this embodiment, gas maintained at a desired temperature Td and desired humidity Hd can be stably supplied to the supply destination (the culture chamber 200). As a result, for example, dew condensation in the culture section and evaporation of culture solution can be suppressed, whereby the concentration of culture solution will be kept constant. Accurate culturing in a space maintained at an optimum humidity can be achieved.

In addition, according to the present invention, the gas (air) is positively mixed with the water vapor to temporarily increase the humidity to 100%. Therefore, compared to devices such as that of Patent Document 1, in which the humidity of the introduced air is directly controlled and regulated to the desired humidity, the necessity of ensuring a sufficient gas/vapor mixing section and performing careful humidification is low. This allows the device for humidification (the moisturizing/mixing unit 6) to be small in size, whereby the entire size of the gas supply device can be decreased. This effect can be enhanced when a mixture acceleration means for accelerating the mixture of the air with the water vapor is provided, for example, by configuring the moisturizing/mixing unit 6 to have a substantially cylindrical shape as in this embodiment.

Further, in this embodiment, regarding the temperature and humidity control of air, the air is subjected only to heating after its humidity is increased to 100%. Therefore, this embodiment is advantageous in that energy loss is reduced compared to cases in which air is cooled after humidification. Furthermore, supplying air through a gas supply pipe 14 as described in this embodiment enables efficient supply of humidified air into a small volume reaction container.

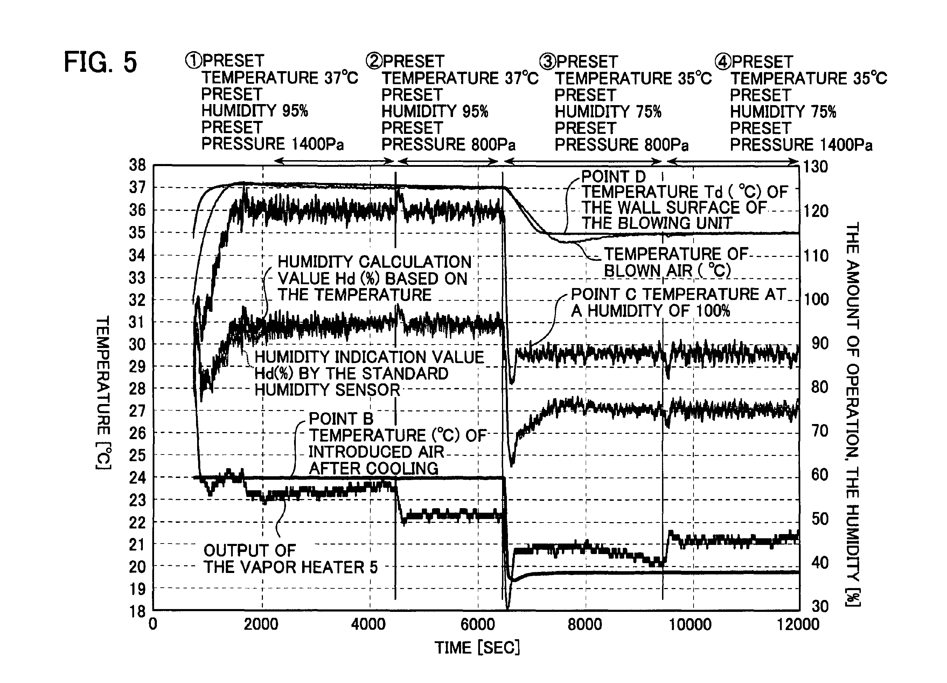

Fig. 5 is a graph illustrating changes in temperature and humidity of air and in the output of the vapor heater 5 when the temperature and humidity control is actually performed by the gas supply device according to this embodiment. After starting control, the target air state is sequentially changed with elapse of time: (1) Td: 37°C, Hd: 95%, set pressure: 1400 Pa; (2) Td: 37°C, Hd: 95%, set pressure: 800 Pa; (3) Td: 35°C, Hd: 75%, set pressure: 800 Pa; (4) Td: 35°C, Hd: 75%, set pressure: 1400 Pa. As shown in Fig. 5, there is a period during which the humidity Hd is unstable immediately after changing the target air state (Td, Hd, pressure). However, both the temperature Td and the humidity Hd are stably controlled after that period. In addition, "humidity change in a case where the humidity of the air is controlled based on detection values of a standard humidity sensor" as described in, for example, Patent Document 1 is also shown in the graph. When the humidity is controlled based on a humidity sensor, the detection values of when the final target humidity Hd is as high as 95% ((1) and (2)), where the accuracy of the humidity sensor remarkably decreases, notably deviate from those of this embodiment in which the humidity of the air is controlled based on detection values of a temperature sensor. It can be seen that this embodiment more accurately controls humidity.

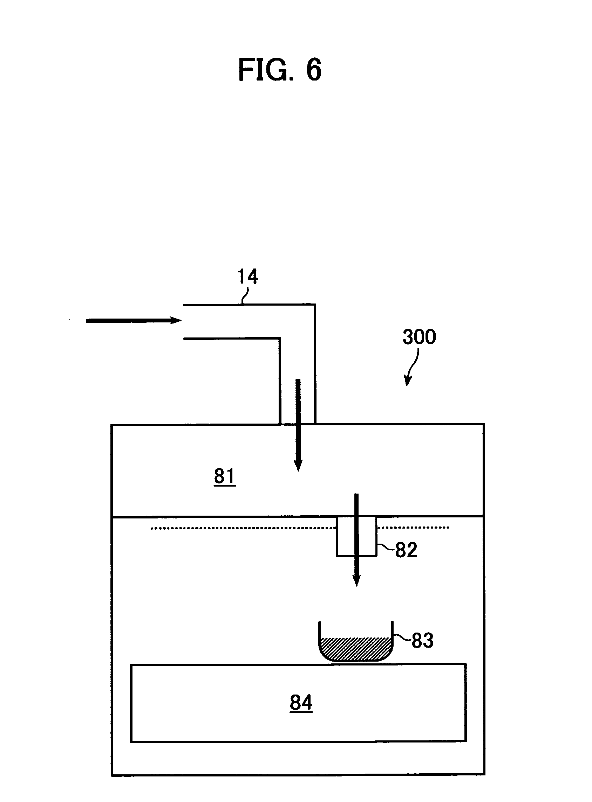

Fig. 6 is a diagram illustrating the configuration of a chemical analysis apparatus having the gas supply device 100 according to the embodiment of the present invention. The chemical analysis apparatus shown in this diagram is such that the temperature inside a reaction chamber 300 is kept constant for a predetermined time period to promote chemical reaction of a sample inside a reaction container 83 and analyze the sample. Therefore, as with the culture apparatus described above, it is necessary to prevent change of the concentration of the sample due to evaporation during analysis.

The chemical analysis apparatus shown in Fig. 6 is provided with the reaction chamber 300 connected to the gas supply pipe 14. Gas maintained at the desired temperature Td and the desired humidity Hd in the gas supply device 100 is introduced into an air buffer 81 inside the reaction chamber 300 through the gas supply pipe 14. The pressure of the gas is regulated in the air buffer 81. The gas is then ejected from one or a plurality of nozzles 82 to one or a plurality of reaction containers 83. Incidentally, the nozzles 82 may be provided to be movable with respect to the air buffer 81 so as to allow the gas ejection position to the reaction container(s) 83 to be changeable. The reaction container(s) 83 is fixed to a base 84, and the base 84 is fixed to or set movable with respect to the reaction chamber 300. When the base 84 is movably set, the reaction container 83 can be transported inside the reaction chamber 300. A sample (reagent and/or specimen solution) is stored in the reaction container 83. Supplying a gas at adjusted temperature and humidity to the reaction container 83 allows the analytical reaction to proceed at a constant temperature while preventing the evaporation from the surface of the solution. Thus, the gas supply device 100 according to the present invention can be applied to chemical analysis apparatuses having a reaction chamber 300. The embodiment of this invention can stably provide gas maintained at a desired temperature Td and desired humidity Hd to the supply destination (the reaction chamber 300). The concentration of the sample can be kept constant, allowing a highly accurate test in a space maintained at optimum humidity.

Incidentally, the above embodiment described a case where the gas subjected to temperature/humidity control is air. However, it is needless to say that, for example, a gas such as carbon dioxide gas may be used as the gas to be conditioned.

Description of Reference Numerals

| | |
|---|---|
| 2 | Fan |
| 3 | Heating/cooling unit |
| 4 | Carburetor |
| 5 | Vapor heater |
| 6 | Moisturizing/mixing unit |
| 7 | Moisture removing unit |
| 8 | Heat applying heater |
| 9 | Temperature maintaining heater |
| 10 | Main controller |
| 10a | Temperature calculation unit |
| 10b | Storage unit |
| 11 | Input unit |
| 12 | Display unit |
| 22 | Temperature sensor (second temperature detection means) |

23    Temperature sensor (first temperature detection means)
41    First fluid level sensor
42    Second fluid level sensor
43    Feed-water pipe
43a   Feed-water pump
43b   Feed-water valve
44    Drain pipe
44a   Drain valve
45    Steam pipe
45a   First drying valve
46    Dew condensation water pipe
46a   Second drying valve
47    Overflow pipe
47a   Overflow valve
47b   U-shaped section
48    Vacuum pipe
48a   Vacuum valve
48b   Vacuum pump
100   Gas supply device
200   Culture chamber
300   Reaction chamber
Hd    Final target humidity
Tb    Initial target temperature
Tc    Intermediate target temperature
Td    Final target temperature


**Claims**

1.  A gas temperature/humidity regulation method for obtaining gas regulated at a desired temperature and desired relative humidity, the method comprising the steps of:

    calculating an intermediate target temperature from the desired temperature and the desired relative humidity, the intermediate target temperature being, assuming a gas containing an amount of water vapor equivalent to that contained in the gas at the desired temperature and the desired relative humidity, the temperature of the gas when its humidity is 100%;
    mixing the gas with water vapor to increase the temperature of the gas to the intermediate target temperature, thereby increasing the relative humidity of the gas to 100%; and
    heating the gas heated to the intermediate target temperature to the desired temperature, thereby maintaining the amount of water vapor therein so as to regulate the relative humidity of the gas to the desired relative humidity.

2.  The gas temperature/humidity regulation method according to claim 1, the method further comprising the steps of:

    calculating an initial target temperature from the intermediate target temperature, the initial target temperature being defined as a temperature of the gas required to easily increase the relative humidity of the gas to 100% in the step of mixing the gas with water vapor; and
    heating or cooling the gas before the step of mixing the gas with water vapor, thereby bringing the temperature of the gas close to the initial target temperature.

3.  A gas supply device for supplying gas regulated at a desired temperature and desired relative humidity, the gas supply device comprising:

    gas supply means for supplying gas to the gas supply device;
    mixing means for mixing the gas from the gas supply means with water vapor to heat the gas, thereby increasing the relative humidity of the gas to 100%;
    vapor generating means for, when a gas containing an amount of water vapor equivalent to that contained in the gas at the desired temperature and the desired relative humidity is assumed, and the temperature of the gas of when its humidity is 100% is defined as an intermediate target temperature, generating an amount of water vapor required to heat the gas to the intermediate target temperature in the mixing means; and heating means for heating the gas discharged from the mixing means, thereby maintaining the amount of water vapor contained in the gas until the temperature of the gas reaches the desired temperature.

4. The gas supply device according to claim 3, further comprising:

first temperature detection means for detecting the temperature of the gas at the outlet of the mixing means; and control means for calculating the intermediate target temperature from the desired temperature and the desired relative humidity, and for adjusting the amount of water vapor generated by the vapor generating means so that the temperature detected by the first temperature detection means nears the intermediate target temperature.

5. The gas supply device according to claim 3, further comprising:

heating and cooling means for, when a temperature of a gas required to easily increase the relative humidity of the gas to 100% by the mixing means is defined as an initial target temperature, heating or cooling the gas to be introduced into the mixing means to the initial target temperature.

6. The gas supply device according to claim 5, further comprising:

second temperature detection means for detecting the temperature of the gas at the inlet of the mixing means; wherein the control means calculates the initial target temperature from the intermediate target temperature, and controls the heating and cooling means so that the temperature detected by the second temperature detection means nears the initial target temperature.

7. The gas supply device according to claim 3, wherein:

the gas supply means adjusts the amount of gas supplied to the gas supply device according to the desired amount of gas.

8. The gas supply device according to claim 3, wherein:

the gas supply means is a turbo fan.

9. The gas supply device according to claim 3, wherein:

the gas supply means supplies the gas into the mixing means in such a manner that the gas crosses water vapor sent from the vapor generating means.

10. The gas supply device according to claim 3, wherein:

the mixing means includes moisture removal means for removing excess moisture from the gas mixed with water vapor.

11. The gas supply device according to claim 3, further comprising:

temperature maintaining means for maintaining the temperature of the gas heated by the heating means at the desired temperature.

12. The gas supply device according to claim 3, further comprising:

drainage means for draining water stored in the vapor generating means; and drying means for drying the vapor generating means by supplying air to the vapor generating means.

13. The gas supply device according to claim 3, further comprising:

pressure reducing means for reducing the pressure inside the vapor generating means.

14. The gas supply device according to claim 3, wherein:

the vapor generating means includes an overflow pipe.

15. A culture apparatus comprising:

the gas supply device according to claim 3; and
a culture chamber into which gas maintained at the desired temperature and the desired humidity is supplied from the gas supply device.

16. A chemical analysis apparatus comprising:

the gas supply device according to claim 3; and
a reaction chamber into which gas maintained at the desired temperature and the desired humidity is supplied from the gas supply device.

# FIG. 1

# FIG. 2

| | | |
|---|---|---|
| INPUT UNIT 11 | DISPLAY UNIT 12 | |

PRESSURE SENSOR (AIR QUANTITY SENSOR) 25 → AIR QUANTITY DETECTION CIRCUIT 51

FIRST FLUIDLEVEL SENSOR 41 → WATER LEVEL DETECTION CIRCUIT 52

SECOND FLUID LEVEL SENSOR 42

TEMPERA-TURE SENSOR 21 → TEMPERATURE DETECTION CIRCUIT 53

TEMPERA-TURE SENSOR 22 → TEMPERATURE DETECTION CIRCUIT 54

TEMPERA-TURE SENSOR 23 → TEMPERATURE DETECTION CIRCUIT 55

TEMPERA-TURE SENSOR 24 → TEMPERATURE DETECTION CIRCUIT 56

TEMPERATURE CALCULATION UNIT 10a

MAIN CONTROLLER

STORAGE UNIT 10b / 10

PUMP DRIVING CIRCUIT 57 → FEEDWATER PUMP 43a

FEEDWATER VALVE DRIVING CIRCUIT 58 → FEEDWATER VALVE 43b

DRAIN VALVE DRIVING CIRCUIT 59 → DRAIN VALVE 44a

FAN DRIVING CIRCUIT 60 → FAN 2

HEATING AND COOLING UNIT DRIVING CIRCUIT 61 → HEATING AND COOLING UNIT 3

VAPOR HEATER DRIVING CIRCUIT 62 → VAPOR HEATER 5

HEAT APPLYING HEATER DRIVING CIRCUIT 63 → HEAT APPLYING HEATER 8

WARM KEEPING HEATER DRIVING CIRCUIT 64 → WARM KEEPING HEATER 9

PUMP DRIVING CIRCUIT 65 → VACUUM PUMP 48b

TIMER 13

15

FIG. 3

START

PROMPT AN OPERATOR TO INPUT FINAL TARGET
TEMPERATURE AND FINAL TARGET HUMIDITY — S101

INPUT THE FINAL TARGET TEMPERATURE
AND THE FINAL TARGET HUMIDITY — S102

CALCULATE INTERMEDIATE TARGET TEMPERATURE Tc — S103

PROMPT THE OPERATOR TO
TURN ON AN OPERATION SWITCH — S104

TURN ON THE OPERATION SWITCH — S105

OPERATE THE FAN — S106

CALCULATE INITIAL TARGET TEMPERATURE Tb — S107

S109A    S108

Tb > Ta    S109B

CLOSE THE DRAIN VALVE
OPEN THE FEEDWATER
VALVE — S113

Y                    N

PERFORM THE
TEMPERATURE
CONTROL OPER
-ATION OF THE
HEATING AND
COOLING UNIT
(HEATING)

PERFORM THE
TEMPERATURE
CONTROL OPER
-ATION OF THE
HEATING AND
COOLING UNIT
(COOLING)

S110

PERFORM THE TEMPERATURE
CONTROL OPERATION OF THE
VAPOR HEATER

S111

PERFORM THE TEMPERATURE
CONTROL OPERATION OF THE
HEAT APPLYING HEATER

S112

PERFORM THE TEMPERATURE
CONTROL OPERATION OF THE
WARM KEEPING HEATER

S114
IS THE
FIRST FLUID
LEVEL SENSOR IN AN
OFF STATE
?

N

S115
Y
TURN ON THE
FEEDWATER PUMP

S116
IS THE
SECOND FLUID
LEVEL SENSOR IN AN
ON STATE
?

N

S117
Y
TURN OFF THE
FEEDWATER PUMP

S118
IS THE
OPERATION SWITCH IN AN OFF
STATE ?

N

Y

A

## FIG. 4

(A)

PROMPT THE OPERATOR TO SELECT WHICH DRYING METHOD IS TO BE USED — S119

**S120**
WHAT DRYING METHOD HAS BEEN SELECTED?

WARM AIR METHOD

VACUUM METHOD

**S121**

| |
|---|
| OPEN THE DRAIN VALVE |
| CLOSE THE FEEDWATER VALVE |
| CONTINUE THE OPERATION OF THE FAN |
| PERFORM THE HEATING OPERATION OF THE HEATING AND COOLING UNIT |
| START THE TIMER |

**S124**

| |
|---|
| OPEN THE DRAIN VALVE |
| CLOSE THE FEEDWATER VALVE |
| CONTINUE THE OPERATION OF THE FAN |
| PERFORM THE HEATING OPERATION OF THE HEATING AND COOLING UNIT |
| START THE TIMER |

**S125** IS TIME UP? — N

**S122** IS TIME UP? — N

Y

**S126**

| |
|---|
| CLOSE THE DRAIN VALVE |
| CLOSE THE DRYING VALVE 1 |
| CLOSE THE DRYING VALVE 2 |
| CLOSE THE OVERFLOW VALVE |
| CLOSE THE FEEDWATER VALVE |
| OPEN THE VACUUM VALVE |

**S127**

| |
|---|
| START THE VACUUM PUMP |
| START THE TIMER |

**S128** IS TIME UP? — N

Y

**S123**

| |
|---|
| STOP THE FAN |
| STOP THE HEATING AND COOLING UNIT |

**S129**

| |
|---|
| STOP THE FAN |
| STOP THE HEATING AND COOLING UNIT |

**S130**

| |
|---|
| STOP THE VACUUM PUMP |

**S131**

| |
|---|
| CLOSE THE VACUUM VALVE |

( END )

FIG. 5

① PRESET
TEMPERATURE 37°C
PRESET
HUMIDITY 95%
PRESET
PRESSURE 1400Pa

② PRESET
TEMPERATURE 37°C
PRESET
HUMIDITY 95%
PRESET
PRESSURE 800Pa

③ PRESET
TEMPERATURE 35°C
PRESET
HUMIDITY 75%
PRESET
PRESSURE 800Pa

④ PRESET
TEMPERATURE 35°C
PRESET
HUMIDITY 75%
PRESET
PRESSURE 1400Pa

POINT D
TEMPERATURE Td ( °C) OF
THE WALL SURFACE OF
THE BLOWING UNIT

TEMPERATURE OF
BLOWN AIR ( °C)

HUMIDITY CALCULATION
VALUE Hd (%) BASED ON
THE TEMPERATURE

POINT C TEMPERATURE AT
A HUMIDITY OF 100%

HUMIDITY INDICATION VALUE
Hd(%) BY THE STANDARD
HUMIDITY SENSOR

POINT B
TEMPERATURE (°C) OF
INTRODUCED AIR
AFTER COOLING

OUTPUT OF
THE VAPOR HEATER 5

TEMPERATURE [°C]

THE AMOUNT OF OPERATION, THE HUMIDITY [%]

TIME [SEC]

EP 2 492 605 A1

# FIG. 6

## INTERNATIONAL SEARCH REPORT

| | International application No. |
|---|---|
| | PCT/JP2010/067931 |

A. CLASSIFICATION OF SUBJECT MATTER
*F24F11/02*(2006.01)i, *F24F6/00*(2006.01)i, *F24F6/18*(2006.01)i

According to International Patent Classification (IPC) or to both national classification and IPC

B. FIELDS SEARCHED

Minimum documentation searched (classification system followed by classification symbols)
F24F11/02, F24F6/00, F24F6/18

Documentation searched other than minimum documentation to the extent that such documents are included in the fields searched
Jitsuyo Shinan Koho          1922-1996   Jitsuyo Shinan Toroku Koho   1996-2011
Kokai Jitsuyo Shinan Koho    1971-2011   Toroku Jitsuyo Shinan Koho   1994-2011

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 4-172507 A  (Tiyoda Manufacturing Co., Ltd.), 19 June 1992 (19.06.1992), claims; page 2, upper right column, line 8 to page 3, lower left column, line 3; fig. 1, 2 (Family: none) | 1-16 |
| Y | JP 7-42971 A  (Takasago Thermal Engineering Co., Ltd.), 10 February 1995 (10.02.1995), claims; paragraphs [0020] to [0032]; fig. 1, 2 (Family: none) | 1-16 |

☐ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 06 January, 2011 (06.01.11) | Date of mailing of the international search report 18 January, 2011 (18.01.11) |
|---|---|
| Name and mailing address of the ISA/ Japanese Patent Office | Authorized officer |
| Facsimile No. | Telephone No. |

Form PCT/ISA/210 (second sheet) (July 2009)

**EP 2 492 605 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 3094154 A